(19) Europäisches Patentamt
European Patent Office
Office européen des brevets

(11) EP 3 217 953 B1

(12) EUROPÄISCHE PATENTSCHRIFT

(45) Veröffentlichungstag und Bekanntmachung des Hinweises auf die Patenterteilung:
**09.01.2019 Patentblatt 2019/02**

(21) Anmeldenummer: **15788066.7**

(22) Anmeldetag: **03.11.2015**

(51) Int Cl.:
*A61K 8/46* (2006.01) *A61K 8/73* (2006.01)
*A61K 8/92* (2006.01) *A61K 8/37* (2006.01)
*A61Q 5/02* (2006.01)

(86) Internationale Anmeldenummer:
**PCT/EP2015/075551**

(87) Internationale Veröffentlichungsnummer:
**WO 2016/074986 (19.05.2016 Gazette 2016/20)**

(54) **SHAMPOOS UND SPÜLUNGEN MIT KONDITIONIERENDER WIRKUNG**

SHAMPOOS AND RINSES WITH CONDITIONING EFFECT

SHAMPOINGS ET RINÇAGES À EFFET REVITALISANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.11.2014 EP 14193046**

(43) Veröffentlichungstag der Anmeldung:
**20.09.2017 Patentblatt 2017/38**

(73) Patentinhaber: **BASF SE**
**67056 Ludwigshafen (DE)**

(72) Erfinder:
- **MAUER, Werner**
**48324 Sendenhorst-Albersloh (DE)**
- **SEIPEL, Werner**
**40723 Hilden (DE)**
- **HAAKE, Hans-Martin**
**40699 Erkrath (DE)**
- **CORNELSEN, Sybille**
**40883 Ratingen (DE)**
- **PELLON, Guadalupe**
**40597 Düsseldorf (DE)**
- **GLASMACHER, Birgit**
**40597 Düsseldorf (DE)**
- **GONDEK, Helga**
**40591 Düsseldorf (DE)**

(74) Vertreter: **BASF IP Association**
**BASF SE**
**G-FLP-C006**
**67056 Ludwigshafen (DE)**

(56) Entgegenhaltungen:
**WO-A1-2014/177292 WO-A2-2008/155073**
**WO-A2-2013/007473 DE-A1-102008 034 388**

- **DATABASE GNPD [Online] MINTEL; 30. Juli 2010 (2010-07-30), L'Occitane: "L'Occitane en Provence, Aromachologie 5 Essential Oils", XP002738731, Database accession no. 1363498**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 217 953 B1

## Beschreibung

Die vorliegende Erfindung betrifft eine Zusammensetzung, die als Shampoo oder Haarspülung geeignet ist, enthaltend mindestens ein Tensid, mindestens ein kationisches Polymer, ethoxylierte Fettsäureglyceride, ethoxyilierte Mono- und Diglycerinester, Trimethylpropan-EO/PO-Trioleat, NaCl und Wasser. Weiterhin betrifft die vorliegende Erfindung ein Zwischenprodukt, das zur Herstellung der genannten Zusammensetzung geeignet ist, enthaltend ethoxylierte Fettsäureglyceride, ethoxyilierte Mono- und Diglycerinester und Trimethylpropan-EO/PO-Trioleat.

Die meisten Shampoos, die aktuell im Markt angetroffen werden, enthalten eine Kombination aus (1) einem anionischen Tensid, (2) einem Cotensid, (3) einem kationischem Polymer und (4) einem oder mehreren so genannten Emollients oder Wachsen. Dabei dienen die Emollients oder Wachse zur Konditionierung der Haare.

Als Cotensid (2) werden verschiedene Tenside verwendet, z. B. Coco-Betain, APG, Amphoacetate. Es werden also unter anderem betainische, nichtionische, und ggf auch anionische Tenside verwendet.

Als kationische Polymere (3) werden unter anderem verwendet Polyquaternium-10 (abgekürzt als PQ 10), kationisch modifizierte Cellulose, kationische Guarderivate.

Als Emollients (4) werden unter anderem native oder synthetische Öle oder Silikone verwendet.

Durch so genannte Koacervatbildung (Koazervation) (das ist die Ausfällung eines Komplexes aus Kationpolymer und Aniontensid) beim Verdünnen (Auswaschen der Haare) werden kationische Polymere und Emollients/Wachse auf dem Haar abgeschieden. Das deponierte Material reduziert dann die Reibung beim Kämmen der nassen und trockenen Haare. Gemessen wird dies als Nass- und Trockenkämmarbeit. Die als Konditioniermittel verwendeten Substanzen sind üblicherweise hydrophob. Häufig werden Silikone (Polysiloxane) als Emollient verwendet.

Unter Haarkonditionierung versteht der Fachmann die Behandlung von Haaren mit pflegenden so genannten Rinse-off-Formulierungen (also Formulierungen, die abgespült werden) oder so genannten Leave-on-Formulierungen (also Formulierungen, die auf den Haaren verbleiben, ohne abgespült zu werden), insbesondere mit pflegenden Shampoos oder Spülungen (engl. Conditioner). Diese Behandlung führt insbesondere zu einer leichteren Kämmbarkeit der Haare im nassen und trockenen Zustand, sowohl in den Längen als auch in den Spitzen (bessere Entwirrbarkeit), zu verbesserten taktilen Eigenschaften wie Glätte, Weichheit und Geschmeidigkeit sowie zu mehr Haarglanz, weniger elektrostatischer Aufladung und besserer Frisierbarkeit. Insgesamt wird somit bei der Konditionierung ein gepflegter und gesund wirkender Gesamtzustand des Haares erzielt.

Neben den oben genannten hydrophoben Substanzen zur Verbesserung der Kämmbarkeit werden hydrophile Komponenten wie z. B. Cetiol® HE (ein Kokosfettsäure-mono- und diglycerid + 7 mol Ethylenoyideinheiten (im Folgenden mit EO abgekürzt)) oder Eumulgin® CO 40 (ein hydriertes Rizinusöl mit 40 mol EO) als Solubilisatoren (das sind Lösevermittler für Öle, hydrophobe Substanzen oder auch Parfums) in Shampoos eingesetzt.

DE 10 2008 034 388 offenbart eine tensidhaltige Zusammensetzung, enthaltend neben einer oder mehrerer Ölkomponente(n) ein Gemisch aus Lösungsvermittlern, die ausgewählt sind aus der Gruppe der a) ethoxylierten Fettalkohole, b) ethoxylierten hydrierten Rizinusöle und c) ethoxylierten Mono-, Di- oder Triglycerinester, dadurch gekennzeichnet, dass das Verhältnis der Komponenten a):b):c) im Bereich von 1:(2-4):(3-4) liegt.

WO 00/64410 beschreibt die Verbesserung der Nasskämmbarkeit durch Verwendung eines Shampoos bestehend aus Tensid plus kationischem Polymer und/oder kationischem Tensid und Poly-alpha-olefin-Öl ausgehend von C6-16 Alken-Monomeren.

WO 2005/048971 beschreibt die Verwendung hydrophiler Solubilisatoren in Formulierungen zur Körperreinigung mit dem Ziel reduzierter Schädigung von Enzymen auf der Haut.

Der vorliegenden Erfindung liegt die Aufgabe zugrunde, zu den bekannten Shampoos oder Spülungen (englisch Conditioner) mit konditionierender Wirkung, die auf hydrophoben Emollients oder Wachsen basieren, eine Alternative bereit zu stellen.

Dabei sollten nach Möglichkeit Shampoos oder Spülungen mit konditionierender Wirkung bereitgestellt werden, die nicht zwingend Silikone enthalten müssen, weil silikonfreie Produkte aktuell am Markt nachgefragt werden.

Außerdem sollte nach Möglichkeit eine gute Konditionierwirkung insbesondere auch an geschädigtem Haar erzielt werden, welches durch die Schädigung hydrophil ist und damit mit hydrophoben Pflegesubstanzen eine weniger starke Wechselwirkung eingeht.

Bei der Suche nach Formulierungen für Shampoos oder Spülungen, die zu verbesserter Kämmbarkeit beitragen, wurde überraschend gefunden, dass eine Kombination von hydrophilen Solubilisatoren mit kationischen Pflegepolymeren und Tensiden auch ohne Verwendung hydrophober Substanzen eine signifikante Verbesserung der Kämmbarkeit zur Folge hatte.

## Zusammensetzung

Die oben genannte Aufgabe wird gelöst durch eine Zusammensetzung, die als Shampoo oder Haarspülung geeignet ist, enthaltend

- mindestens ein Tensid,
- optional mindestens ein Cotensid, welches von dem mindestens einem Tensid verschieden ist,
- mindestens ein kationisches Polymer,
- ethoxylierte Fettsäureglyceride,
- ethoxyilierte Mono- und Diglycerinester,
- Trimethylpropan-EO/PO-Trioleat,
- NaCl
- optional Glycerin,
- optional weitere in Shampoos oder Haarspülungen übliche Inhaltsstoffe, und
- Wasser.

Diese Zusammensetzung ist ein Gegenstand der vorliegenden Erfindung.

**Tenside**

Das in der erfindungsgemäßen Zusammensetzung enthaltene mindestens eine Tensid kann ein beliebiges Tensid sein. Insbesondere kommt in Betracht ein Tensid ausgewählt aus der Gruppe bestehend aus einem Sulfat, einem ethoxilierten Sulfat, einem Sulfonat, einem Alkylpolyglycosid, einem Derivat eines Alkylpolyglycosids, einem Betain, einem Amphoacetat, einem Glutamat, einem Sulfosuccinat, einem Taurat, einem Glycinat und einem Isethionat. In einer Ausführungsform der vorliegenden Erfindung ist das mindestens eine Tensid ein anionisches Tensid, bevorzugt ein Natriumalkylethersulfat mit 12 bis 14 C-Atomen und zwei Ethylenoxieinheiten als Etherkomponente.

In einer weiteren Ausführungsform der vorliegenden Erfindung ist das mindestens eine Tensid ein anionisches und/oder nichtionisches und/oder amphoteres und/oder zwitterionisches Tensid, dessen Anteil in den erfindungsgemäßen Zusammensetzungen bevorzugt bei 3 bis 40 Gew.-%, vorzugsweise 5 bis 35 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, $\alpha$-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen.

Das in der erfindungsgemäßen Zusammensetzung enthaltene mindestens eine Tensid ist bevorzugt in einer Menge von 10 bis 20 Gew.-% in der erfindungsgemäßen Zusammensetzung enthalten.

**Cotenside**

Das in der erfindungsgemäßen Zusammensetzung enthaltene mindestens eine Cotensid kann ein beliebiges Cotensid sein. Es ist so auszuwählen, dass es von dem mindestens eine Tensid verschieden ist. In einer Ausführungsform der vorliegenden Erfindung ist das mindestens eine Tensid ein Cocamidopropylbetain.

In einer Ausführungsform enthält die Zusammensetzung gemäß der vorliegenden Erfindung eine Kombination aus einem Natriumalkylethersulfat mit 12 bis 14 C-Atomen und zwei Ethylenoxieinheiten als Etherkomponente als Tensid und aus einem Cocamidopropylbetain als Cotensid.

Das in der erfindungsgemäßen Zusammensetzung enthaltene mindestens eine Cotensid ist bevorzugt in einer Menge von 10 bis 15 Gew.-% in der erfindungsgemäßen Zusammensetzung enthalten.

**Kationische Polymere**

Das in der erfindungsgemäßen Zusammensetzung enthaltene mindestens eine kationische Polymer kann ein

beliebiges kationisches Polymer sein. In einer Ausführungsform der vorliegenden Erfindung ist das mindestens eine kationische Polymer ausgewählt aus der Gruppe bestehend aus einem kationisch modifizierten Cellulosederivat, PQ 10, PQ 67, einem kationisch modifizierte Guarderivat, Guar Hydroxypropyltrimonium Chlorid, einem kationischen Homo- oder Copolymer auf der Basis von Acrylamid, einem kationischen Homo- oder Copolymer auf der Basis von Vinylpyrrolidon, einem kationischen Homo- oder Copolymer auf der Basis von quaternisiertem Vinylimidazol und einem kationischen Homo- oder Copolymer auf der Basis von Methacrylaten.

Das in der erfindungsgemäßen Zusammensetzung enthaltene mindestens eine kationische Polymer ist bevorzugt in einer Menge von 0,02 bis 5 Gew.-% in der erfindungsgemäßen Zusammensetzung enthalten.

Geeignete kationische Polymere sind weiterhin beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polygly-colen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationi-sche Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR-A 2252840 sowie deren vernetzte wasserlöslichen Polymere, kationische Chitinderivate, wie beispiels-weise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Kationpolymere werden in den erfindungsgemäßen Zusammensetzungen vorzugsweise in Mengen von 0,02 bis 5 Gew. %, vorzugsweise 0,05 bis 3 Gew.% und besonders bevorzugt in Mengen von 0,1 bis 2 Gew. % eingesetzt.

**Ethoxylierte Fettsäureglyceride**

Die in der erfindungsgemäßen Zusammensetzung enthaltenen ethoxyilierten Fettsäureglyceride sind ein Gemisch aus mehreren Einzelverbindungen. Sie sind erhältlich durch die Umsetzung von Fettsäureglyceriden mit Ethylenoxid (EO) im Alkalischen, z. B. in Gegenwart von KOH, bei erhöhter Temperatur, z. B. 100 bis 150 °C, wobei Umesterungen und Ethoxylierungen stattfinden, so dass das resultierende Produkt auch ethoxylierte Partialglyceride enthalten kann. In einer Ausführungsform der vorliegenden Erfindung wird ethoxiliertes, hydrierte Rizinusöle eingesetzt. In einer Ausführungsform der vorliegenden Erfindung wird ethoxiliertes, hydriertes Rizinusöl mit 40 Ethylenoxideinheiten eingesetzt.

Als Fettsäure werden im Zusammenhang mit der vorliegenden Erfindung insbesondere aliphatische Monocarbonsäuren mit unverzweigter Kohlenstoffkette, insbesondere solche mit 6 bis 30 C-Atomen bezeichnet.

Die in der erfindungsgemäßen Zusammensetzung enthaltenen ethoxyilierten Fettsäureglyceride sind bevorzugt in einer Menge von 0,06 bis 3,5 Gew.-%, bevorzugt 0,12 bis 2,1 Gew.-%, in der erfindungsgemäßen Zusammensetzung enthalten.

**Ethoxyilierte Mono- und Diglycerinester**

Die in der erfindungsgemäßen Zusammensetzung enthaltenen ethoxyilierten Mono- und Diglycerinester sind ein Gemisch aus mehreren Einzelverbindungen. Sie sind Gemische von Molekülen, die je einen Glycerinrest enthalten, und die je einen oder zwei Fettsäurereste enthalten, und die jeweils im statistischen Mittel eine bestimmte Anzahl an Ethylenoxidresten enthalten. In einer Ausführungsform der vorliegenden Erfindung werden ethoxilierte Kokosöl-Partialglyceride verwendet. In einer Ausführunsgform der vorliegenden Erfindung werden ethoxilierte Kokosöl-Partialglyceride mit 7 Ethylenoxideinheiten verwendet.

Die in der erfindungsgemäßen Zusammensetzung enthaltenen ethoxyilierten Mono- und Diglycerinester sind bevorzugt in einer Menge von 0,06 bis 2,5 Gew.-%, bevorzugt 0,12 bis 1,5 Gew.-%, in der erfindungsgemäßen Zusammensetzung enthalten.

**Trimethylpropan-EO/PO-Trioleat**

Das in der erfindungsgemäßen Zusammensetzung enthaltene Trimethylpropan-EO/PO-Trioleat ist ein Gemisch aus mehreren Einzelverbindungen. Es ist erhältlich durch die Umsetzung von Trimethylolpropantrioleat mit Ethylenoxid und Propylenoxid unter alkalischen Bedingungen. Dabei findet, zumindest zum Teil, eine Einlagerung der Ethylenoxideinheiten (EO) und der Propylenoxideinheiten (PO) in die Estergruppen des Trimethylolpropantrioleat statt. Das Trimethylpropan-EO/PO-Trioleat wird charakterisiert durch seinen Gehalt an EO- und PO-Einheiten pro Molekül im

statistischen Mittel. In einer Ausführunsgform der vorliegenden Erfindung wird Trimethylpropan-EO/PO-Trioleat, mit 120 Ethylenoxideinheiten (EO) und 10 Propylenoxideinheiten (PO) eingesetzt.

Das in der erfindungsgemäßen Zusammensetzung enthaltene Trimethylpropan-EO/PO-Trioleat ist bevorzugt in einer Menge von 0,03 bis 0,5 Gew.-%, bevorzugt 0,06 bis 0,3 Gew.-%, in der erfindungsgemäßen Zusammensetzung enthalten.

**NaCl (Kochsalz)**

Ein Merkmal der erfindungsgemäßen Zusammensetzung ist die Anwesenheit von NaCl. Dieses gewährleistet, dass der Koacervatmechanismus aus anionischem Tensid und kationischem Polymer in der Verdünnungsphase initiiert wird. Dabei kann das NaCl durch das Cotensid (beispielsweise Cocamidopropylbetain, welches häufig bereits herstellungsbedingt ca. 5-7,5 Gew.-% NaCl enthält) mit eingeschleppt und/oder separat zugegeben werden.

**Wachse (auch Wachskörper genannt)**

Als Wachskörper kommen in den Wachsdispersionen in Frage: Alkylenglycolester, Fettsäurealkanolamide, Partialglyceride, Ester von mehrwertigen, gegebenenfalls hydroxysubstituierten Carbonsäuren, Fettalkohole, Fettketone, Fettaldehyde, Fettether, Fettcarbonate, Ringöffnungsprodukte von Olefinepoxiden sowie deren Mischungen.

Bei den **Alkylenglycolestern,** handelt es sich üblicherweise um Mono- und/oder Diester von Alkylenglycolen, die der Formel **(I)** folgen,

$$R^1CO(OA)_nOR^2 \qquad (I)$$

in der $R^1CO$ für einen linearen oder verzweigten, gesättigten oder ungesättigten Acylrest mit 6 bis 22 Kohlenstoffatomen, $R^2$ für Wasserstoff oder $R^1CO$ und A für einen linearen oder verzweigten Alkylenrest mit 2 bis 4 Kohlenstoffatomen und n für Zahlen von 1 bis 5 steht. Typische Beispiele sind Mono- und/oder Diester von Ethylenglycol, Propylenglycol, Diethylenglycol, Dipropylenglycol, Triethylenglycol oder Tetraethylenglycol mit Fettsäuren mit 6 bis 22, vorzugsweise 12 bis 18 Kohlenstoffatomen als da sind: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Ethylenglycolmono- und/oder -distearat.

Andere Wachskörper wie **Fettsäurealkanolamide,** folgen der Formel **(II),**

$$R^3CO\text{-}NR^4\text{-}B\text{-}OH \qquad (II)$$

in der $R^3CO$ für einen linearen oder verzweigten, gesättigten oder ungesättigten Acylrest mit 6 bis 22 Kohlenstoffatomen, $R^4$ für Wasserstoff oder einen gegebenenfalls hydroxysubstituierten Alkylrest mit 1 bis 4 Kohlenstoffatomen und B für eine lineare oder verzweigte Alkylengruppe mit 1 bis 4 Kohlenstoffatomen steht. Typische Beispiele sind Kondensationsprodukte von Ethanolamin, Methylethanolamin, Diethanolamin, Propanolamin, Methylpropanolamin und Dipropanolamin sowie deren Mischungen mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Stearinsäureethanolamid.

**Partialglyceride,** stellen Mono und/oder Diester des Glycerins mit linearen, gesättigten und/oder partiell ungesättigten Fettsäuren, beispielsweise Capronsäure, Caprylsäure, Caprinsäure, Laurinsäure, Myristinsäure, Palmitinsäure, Palmoleinsäure, Talgfettsäure, Stearinsäure, Behensäure sowie deren technische Mischungen dar. Sie folgen der Formel **(III),**

$$CH_2O(CH_2CH_2O)_x\text{-}COR^5$$

|

$$CH\text{-}O(CH_2CH_2O)_yR^6 \qquad \text{(III)}$$

|

$$CH_2O(CH_2CH_2O)_z\text{-}R^7$$

in der $R^5CO$ für einen Acylrest mit 6 bis 22 Kohlenstoffatomen, vorzugsweise für einen linearen, gesättigten Acylrest mit 6 bis 22 Kohlenstoffatomen, $R^6$ und $R^7$ unabhängig voneinander für Wasserstoff oder $R^5CO$, x, y und z in Summe für 0 oder für Zahlen von 1 bis 30 und X für ein Alkali- oder Erdalkalimetall mit der Maßgabe steht, daß mindestens einer der beiden Reste $R^6$ und $R^7$ Wasserstoff darstellt. Typische Beispiele sind Laurinsäuremonoglycerid, Laurinsäurediglycerid, Kokosfettsäuremonoglycerid, Kokosfettsäuretriglycerid, Palmitinsäuremonoglycerid, Palmitinsäuretriglycerid, Stearinsäuremonoglycerid, Stearinsäurediglycerid, Talgfettsäuremonoglycerid, Talgfettsäurediglycerid, Behensäuremonoglycerid, Behensäurediglycerid sowie deren technische Gemische, die untergeordnet aus dem Herstellungsprozeß noch geringe Mengen an Triglycerid enthalten können.

Als Wachskörper kommen weiterhin als bevorzugte Gruppe **Ester von mehrwertigen, gegebenenfalls hydroxysubstituierten Carbonsäuren** mit Fettalkoholen mit 6 bis 22 Kohlenstoffatomen in Frage. Als Säurekomponente dieser Ester kommen beispielsweise Malonsäure, Maleinsäure, Fumarsäure, Adipinsäure, Sebacinsäure, Azelainsäure, Dodecandisäure, Phthalsäure, Isophthalsäure und insbesondere Bernsteinsäure sowie Äpfelsäure, Citronensäure und insbesondere Weinsäure und deren Mischungen in Betracht. Die Fettalkohole enthalten 6 bis 22, vorzugsweise 12 bis 18 und insbesondere 16 bis 18 Kohlenstoffatome in der Alkylkette. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Die Ester können als Voll oder Partialester vorliegen, vorzugsweise werden Mono- und vor allem Diester der Carbon- bzw. Hydroxycarbonsäuren eingesetzt. Typische Beispiele sind Bernsteinsäuremono- und -dilaurylester, Bernsteinsäuremono- und -dicetearlyester, Bernsteinsäuremono- und -distearylester, Weinsäuremono- und -dilaurylester, Weinsäuremono- und dikokosalkylester, Weinsäuremono- und -dicetearylester, Citronensäuremono-, -di- und -trilaurylester, Citronensäuremono-, -di- und-trikokosalkylester sowie Citronensäuremono-, -di- und -tricetearylester.

Als dritte bevorzugte Gruppe von Wachskörpern können **Fettalkohole** eingesetzt werden, die der Formel **(IV)** folgen,

$$R^8OH \qquad \text{(IV)}$$

in der $R^8$ für einen linearen, gegebenenfalls hydroxysubstituierten Alkylrest und/oder Acylrest mit 16 bis 48, vorzugsweise 18 bis 36 Kohlenstoffatomen steht. Typische Beispiele für geeignete Alkohole sind Cetearylalkohol, Hydroxystearylalkohol, Behenylalkohol sowie Oxidationsprodukte langkettiger Paraffin.

**Fettketone,** die als Komponente in Betracht kommen, folgen vorzugsweise der Formel **(V),**

$$R^9\text{-}CO\text{-}R^{10} \qquad \text{(V)}$$

in der $R^9$ und $R^{10}$ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Die Ketone können nach Verfahren des Stands der Technik hergestellt werden, beispielsweise durch Pyrolyse der entsprechenden Fettsäure-Magnesiumsalze. Die Ketone können symmetrisch oder unsymmetrisch aufgebaut sein, vorzugsweise unterscheiden sich die beiden Reste $R^{13}$ und $R^{14}$ aber nur um ein Kohlenstoffatom und leiten sich von Fettsäuren mit 16 bis 22 Kohlenstoffatomen ab.

Als Wachskörper geeignete **Fettaldehyde** entsprechen vorzugsweise der Formel **(VI),**

$$R^{11}COH \qquad \text{(VI)}$$

in der $R^{11}CO$ für einen linearen oder verzweigten Acylrest mit 24 bis 48, vorzugsweise 28 bis 32 Kohlenstoffatomen steht.

Ebenso kommen **Fettether** vorzugsweise der Formel **(VII)** in Frage,

$$R^{12}\text{-O-}R^{13} \qquad \textbf{(VII)}$$

in der $R^{12}$ und $R^{13}$ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Fettether der genannten Art werden üblicherweise durch saure Kondensation der entsprechenden Fettalkohole hergestellt. Fettether mit besonders vorteilhaften Perlglanzeigenschaften werden durch Kondensation von Fettalkoholen mit 16 bis 22 Kohlenstoffatomen, wie beispielsweise Cetylalkohol, Cetearylalkohol, Stearylalkohol, Isostearylalkohol, Oleylalkohol, Behenylalkohol und/oder Erucylalkohol erhalten.

Als Komponente kommen weiterhin **Fettcarbonate** vorzugsweise der Formel **(VIII)** in Betracht,

$$R^{14}\text{O-CO-O}R^{15} \qquad \textbf{(VIII)}$$

in der $R^{14}$ und $R^{15}$ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Die Stoffe werden erhalten, indem man beispielsweise Dimethyl- oder Diethylcarbonat mit den entsprechenden Fettalkoholen in an sich bekannter Weise umestert. Demzufolge können die Fettcarbonate symmetrisch oder unsymmetrisch aufgebaut sein. Vorzugsweise werden jedoch Carbonate eingesetzt, in denen $R^{14}$ und $R^{15}$ gleich sind und für Alkylreste mit 16 bis 22 Kohlenstoffatomen stehen. Besonders bevorzugt sind Umesterungsprodukte von Dimethyl- bzw. Diethylcarbonat mit Cetylalkohol, Cetearylalkohol, Stearylalkohol, Isostearylalkohol, Oleylalkohol, Behenylalkohol und/ oder Erucylalkohol in Form ihrer Mono- und Diester bzw. deren technischen Mischungen.

Bei den **Epoxidringöffnungsprodukten** handelt es sich um bekannte Stoffe, die üblicherweise durch säurekatalysierte Umsetzung von endständigen oder innenständigen Olefinepoxiden mit aliphatischen Alkoholen hergestellt werden. Die Reaktionsprodukte folgen vorzugsweise der Formel **(IX),**

**OH**

**|**

**$R^{16}$-CH-CH-$R^{17}$** **(IX)**

**|**

**$OR^{18}$**

in der $R^{16}$ und $R^{17}$ für Wasserstoff oder einen Alkylrest mit 10 bis 20 Kohlenstoffatomen steht, mit der Maßgabe, daß die Summe der Kohlenstoffatome von $R^{16}$ und $R^{17}$ im Bereich von 10 bis 20 liegt und $R^{18}$ für einen Alkyl- und/oder Alkenylrest mit 12 bis 22 Kohlenstoffatomen und/oder den Rest eines Polyols mit 2 bis 15 Kohlenstoffatomen und 2 bis 10 Hydroxylgruppen steht. Typische Beispiele sind Ringöffnungsprodukte von $\alpha$-Dodecenepoxid, $\alpha$-Hexadecenepoxid, $\alpha$-Octadecenepoxid, $\alpha$-Eicosen-epoxid, $\alpha$-Docosenepoxid, i-Dodecenepoxid, i-Hexadecenepoxid, i-Octadecenepoxid, i-Eicosenepoxid und/ oder i-Docosenepoxid mit Laurylalkohol, Kokosfettalkohol, Myristylalkohol, Cetylalkohol, Cetearylalkohol, Stearylalkohol, Isostearylalkohol, Oleylalkohol, Elaidylalkohol, Petroselinylalkohol, Linolylalkohol, Linolenylalkohol, Behenylalkohol und/oder Erucylalkohol. Vorzugsweise werden Ringöffnungsprodukte von Hexa- und/oder Octadecenepoxiden mit Fettalkoholen mit 16 bis 18 Kohlenstoffatomen eingesetzt. Werden anstelle der Fettalkohole Polyole für die Ringöffnung eingesetzt, so handelt es sich beispielsweise um folgende Stoffe: Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin.

**Weitere in Shampoos oder Haarspülungen übliche Inhaltsstoffe**

Als weitere in Shampoos oder Haarspülungen übliche Inhaltsstoffe kommen in Betracht Emulgatoren, Konsistenzgeber, Verdickungsmittel, Polymere, Lecithine, Phospholipide, biogene Wirkstoffe, Antischuppenmittel, Hydrotrope, Filmbildner, Konservierungsmittel, Parfümöle und Farbstoffe.

**Weitere Ausführungsformen der Zusammensetzung**

In einer Ausführungsform enthält die Zusammensetzung gemäß der vorliegenden Erfindung

- mindestens ein anionisches Tensid, bevorzugt ein Natriumalkylethersulfat mit 12 bis 14 C-Atomen und zwei Ethylenoxieinheiten als Etherkomponente,
- optional mindestens ein Cotensid, welches von dem mindestens einem Tensid verschieden ist, und welches ein Cocamidopropylbetain ist,
- mindestens ein kationisches Polymer,
- ethoxilierte, hydrierte Rizinusöle,
- ethoxilierte Kokosöl-Partialglyceride,
- Trimethylpropan-EO/PO-Trioleat,
- NaCl,
- optional Glycerin,
- optional weitere in Shampoos oder Haarspülungen übliche Inhaltsstoffe, und
- Wasser.

In einer Ausführungsform enthält die Zusammensetzung gemäß der vorliegenden Erfindung
10 bis 20 Gew.-% des mindestens einen Tensids,
0 bis 15 Gew.-% des mindestens einen Cotensids,
0,1 bis 5 Gew.-% des kationischen Polymers,
0,06 bis 3,5 Gew.-%, bevorzugt 0,12 bis 2,1 Gew.-%, ethoxylierte Fettsäureglyceride,
0,06 bis 2,5 Gew.-%, bevorzugt 0,12 bis 1,5 Gew.-%, ethoxyilierte Mono- und Diglycerinester,
0,03 bis 0,5 Gew.-%, bevorzugt 0,06 bis 0,3 Gew.-%, Trimethylpropan-EO/PO-Trioleat,
0,1 bis 5 Gew.-%, insbesondere 0,3 bis 3 Gew.-%, NaCl,
optional Glycerin,
optional weitere in Shampoos oder Haarspülungen übliche Inhaltsstoffe enthält, und zur Ergänzung auf 100 Gew.-% Wasser.

In einer Ausführungsform enthält die Zusammensetzung gemäß der vorliegenden Erfindung weniger als 2 Gew.-%, insbesondere weniger als 1 Gew.-%, insbesondere weniger als 0,5 Gew.-% Polysiloxane.
In einer Ausführungsform enthält die Zusammensetzung gemäß der vorliegenden Erfindung weniger als 2 Gew.-%, insbesondere weniger als 1 Gew.-%, insbesondere weniger als 0,5 Gew.-% an Stoffen, die Emollients oder Wachse sind.

**Zwischenprodukt**

Ein weiterer Gegenstand der vorliegenden Erfindung ist ein Zwischenprodukt, das zur Herstellung der erfindungsgemäßen Zusammensetzung geeignet ist, enthaltend

- ethoxylierte Fettsäureglyceride,
- ethoxyilierte Mono- und Diglycerinester,
- Trimethylpropan-EO/PO-Trioleat,
- NaCl
- optional Wasser, und
- optional Glycerin.

Dabei haben die Begriffe ethoxylierte Fettsäureglyceride, ethoxyilierte Mono- und Diglycerinester, und Trimethylpropan-EO/PO-Trioleat dieselbe Bedeutung wie im Zusammenhang mit der erfindungsgemäßen Zusammensetzung und können insbesondere in denjenigen Ausführungsformen verwirklicht werden, wie sie in den Ausführungsformen der erfindungsgemäßen Zusammensetzung verwirklicht werden.

**Mengenanteile im Zwischenprodukt**

In einer Ausführungsform sind die Komponenten des Zwischenprodukts ((a) ethoxylierte Fettsäureglyceride, (b) ethoxyilierte Mono- und Diglycerinester, (c) Trimethylpropan-EO/PO-Trioleat) in einem Massenverhältnis von a:b:c = 2-1:1-2:0,2-0,5, insbesondere 2:1:0,3, in dem Zwischenprodukt vorhanden.

Die in dem erfindungsgemäßen Zwischenprodukt enthaltenen ethoxyilierten Fettsäureglyceride sind bevorzugt in einer Menge von 12 bis 70 Gew.-%, insbesondere 40 bis 66 Gew.-%, insbesondere 55 bis 56 Gew.-%, in dem erfindungsgemäßen Zwischenprodukt enthalten.
Die in dem erfindungsgemäßen Zwischenprodukt enthaltenen ethoxyilierten Mono- und Diglycerinester sind bevorzugt in einer Menge von 12 bis 50 Gew.-%, insbesondere 20 bis 33 Gew.-%, insbesondere 27 bis 28 Gew.-%, in dem erfindungsgemäßen Zwischenprodukt enthalten.
Das in dem erfindungsgemäßen Zwischenprodukt enthaltene Trimethylpropan-EO/PO-Trioleat ist bevorzugt in einer Menge von 6 bis 10 Gew.-%, insbesondere 8 bis 9 Gew.-%, in dem erfindungsgemäßen Zwischenprodukt enthalten.
Das in dem erfindungsgemäßen Zwischenprodukt optional enthaltene Glycerin ist bevorzugt in einer Menge von 3 bis 5 Gew.-% in dem erfindungsgemäßen Zwischenprodukt enthalten.
Das in dem erfindungsgemäßen Zwischenprodukt enthaltene Wasser ist bevorzugt in einer Menge von 6 bis 10 Gew.-%, insbesondere 4 bis 5 Gew.-%, in dem erfindungsgemäßen Zwischenprodukt enthalten.

**Weitere Ausführungsformendes Zwischenprodukts**

Eine Ausführungsform der vorliegenden Erfindung ist das Zwischenprodukt, das zur Herstellung der erfindungsgemäßen Zusammensetzung geeignet ist, enthaltend

- ethoxilierte, hydrierte Rizinusöle,
- ethoxilierte Kokosöl-Partialglyceride,
- Trimethylpropan-EO/PO-Trioleat,
- NaCl
- optional Wasser, und
- optional Glycerin.

Eine weitere Ausführungsform der vorliegenden Erfindung ist das Zwischenprodukt, das zur Herstellung der erfindungsgemäßen Zusammensetzung geeignet ist, enthaltend

- 12 bis 70 Gew.-%, bevorzugt 40 bis 66 Gew.-%, ethoxylierte Fettsäureglyceride, bevorzugt ethoxilierte, hydrierte Rizinusöle,
- 12 bis 50 Gew.-%, bevorzugt 20 bis 33 Gew.-%, ethoxyilierte Mono- und Diglycerinester, bevorzugt ethoxilierte Kokosöl-Partialglyceride,
- 6 bis 10 Gew.-% Trimethylpropan-EO/PO-Trioleat,
- NaCl
- optional Wasser, und
- optional Glycerin.

Eine weitere Ausführungsform der vorliegenden Erfindung ist das Zwischenprodukt, das zur Herstellung der erfindungsgemäßen Zusammensetzung geeignet ist, enthaltend

- 55 bis 56 Gew.-% ethoxiliertes, hydriertes Rizinusöl mit 40 Ethylenoxideinheiten,
- 27 bis 28 Gew.-% ethoxilierte Kokosöl-Partialglyceride mit 7 Ethylenoxideinheiten,
- 8 bis 9 Gew.-% Trimethylpropan-EO/PO-Trioleat, mit 120 Ethylenoxideinheiten (EO) und 10 Propylenoxideinheiten (PO),
- NaCl
- 4 bis 5 Gew.-% Wasser, und
- 3 bis 5 Gew.-% Glycerin.

Üblicherweise wird das Zwischenprodukt der erfindungsgemäßen Zusammensetzung in einer Menge von 0,5 bis 5 Gew.-%, bevorzugt 1 bis 3 Gew.-% zugegeben.

## Weitere Gegenstände der Erfindung

Ein weiterer Gegenstand der vorliegenden Erfindung ist die Verwendung des erfindungsgemäßen Zwischenproduktes zur Herstellung der erfindungsgemäßen Zusammensetzung.

Ein weiterer Gegenstand der vorliegenden Erfindung ist ein Verfahren zur Herstellung der erfindungsgemäßen Zusammensetzung umfassend das Bereitstellen des erfindungsgemäßen Zwischenproduktes und das in Kontakt Bringen des Zwischenproduktes mit den übrigen Bestandteilen der Zusammensetzung.

Ein weiterer Gegenstand der vorliegenden Erfindung ist die Verwendung der erfindungsgemäßen Zusammensetzung zur Konditionierung von Haaren, bevorzugt von menschlichen Haaren.

Ein weiterer Gegenstand der vorliegenden Erfindung ist die Verwendung der erfindungsgemäßen Zusammensetzung zur Verbesserung der Kämmbarkeit, bevorzugt zur Verbesserung der Nasskämmbarkeit, von Haaren, bevorzugt von menschlichen Haaren. Eine besondere Ausführungsform dieser Verwendung ist gegeben, wenn die Haare geschädigt und hydrophil sind.

## Beispiele

Wenn nichts anderes angegeben ist, bedeutet % im folgenden Gewichts-%.
Wenn nichts anderes angegeben ist, bedeutet RT oder Raumtemperatur im Folgenden 20° C.
EO bedeutet Ethylenoxy-Einheiten.

Beschreibung der in den folgenden Beispielen verwendeten Markenprodukte gemäß INCI:

| | |
|---|---|
| Texapon® N 70 | Sodium Laureth Sulfate + 2 EO |
| Dehyton® PK 45 | Cocamidopropyl Betaine |
| Polymer JR 400 | Polyquaternium-10 |
| Arlypon® TT liquid | Trimethylolpropan + 120 EO/10 PO - Random - tri - Oleat |
| Cetiol® HE | Coco, Mono- and Diglyceride + 7 mol EO |
| Eumulgin® CO 40 | hydrogenated Castor Oil + 40 mol EO |
| Dehyquart® CC7 BZ | Polyquaternium-7 |
| Dehyquart® Guar N | Guar Hydroxypropyltrimonium Chloride |
| Dehyquart® Guar TC | Guar Hydroxypropyltrimonium Chloride |
| Dehyquart® Guar HP | Guar Hydroxypropyltrimonium Chloride |
| Euperlan® PK 710 Benz | Glycol Distearate and Sodium Laureth Sulfate and Cocamide MEA |

Es wurden Schampoos gemäß der in der folgenden Tabelle angegebenen Zusammensetzung in Gew.-% hergestellt.

| **Beispiele 12-278-** | **04** | **05** | **06** | **07** | **08** |
|---|---|---|---|---|---|
| **Texapon® N 70** | 15,70 | 15,70 | 15,70 | 15,70 | 15,70 |
| **Dehyton® PK 45** | 7,40 | 7,40 | 7,40 | 7,40 | 7,40 |
| **Polymer® JR 400** | 0,50 | 0,50 | 0,50 | 0,50 | 0,50 |
| **Arlypon® TT liquid** | - | 0,30 | 0,30 | 0,30 | - |
| **Cetiol® HE** | - | - | 1,0 | 1,0 | 1,0 |
| **Eumulgin® CO 40** | - | - | - | 2,0 | 2,0 |
| **Parfum Cotton Touch** | 0,20 | 0,20 | 0,20 | 0,20 | 0,20 |
| **Na-Benzoat** | 0,50 | 0,50 | 0,50 | 0,50 | 0,50 |
| **Zitronensäure 50 % in Wasser** | 0,90 | 0,87 | 0,97 | 0,77 | 0,90 |
| **NaCl** | 0,70 | 0,70 | 0,70 | 0,70 | 0,70 |
| **Wasser** | 74,10 | 73,83 | 72,73 | 70,93 | 71,10 |
| **pH-Wert** | 4,73 | 4,70 | 4,74 | 4,70 | 4,87 |
| **Viskosität bei RT [mPas] *** | 5000 | 25000 | 36000 | 4400 | 600 |

(fortgesetzt)

| Beispiele 12-278- | 04 | 05 | 06 | 07 | 08 |
|---|---|---|---|---|---|
| NK % ** | 87+-10 | 72+-4 | 60+-9 | 39+-4 | 45+-5 |
| * Die Viskosität der erhaltenen Schampoos wurde mittels eines Brookfield Viskosimerters Typ RTV DV-II bei 20 °C gemessen.<br>** Die Nasskämmarbeit in % des Referenzwertes (NK %) wurde gemäß der folgenden Methode bestimmt. | | | | | |

Die Messungen wurden jeweils an 10 Haarsträhnen in einem automatisierten System zur Bestimmung der Nasskämmarbeit durchgeführt.
Die Vorbehandlung der Haarsträhnen (12cm/1g) der Firma IHIP wurden in einem automatisierten Haarbehandlungssystem in folgenden Schritten durchgeführt:

- 30 min Reinigung mit 6 % Natriumlaurylethersulfat, pH 6.5, danach intensives Spülen der Haare,
- 20 min Bleiche mit einer Lösung von 5 % Wasserstoffperoxid, pH 9.4 (mit Ammoniumhydroxidlösung eingestellt), danach intensives Spülen der Haare,
- 30 min Trocknung in einem Luftstrom bei 68° C.

Direkt vor der Nullmessung wurden die Haare für 30 Minuten in Wasser gequollen und anschließend mit einer automatischen Nassauskämmapparatur für 1 Minute ausgespült. Im automatisierten System zur Bestimmung der Nass- und Trockenkämmarbeit wurden die Kämmkräfte während 20 Kämmungen bestimmt und die Kämmarbeit durch Integration der gemessenen Kraft-Weg-Kurven errechnet.

Nach der Nullmessung wurden die Haare sofort mit der Formulierung behandelt (0,25g/g Haar). Nach 5 Minuten Einwirkzeit wurde mit der automatischen Nassauskämmapparatur unter Standardbedingungen (38°C, 1l/Minute) gespült. Die Behandlung und das folgende Ausspülen wurde ein zweites Mal wiederholt. Dann erfolgte die Vergleichsmessung (zur Nullmessung). Die Messungen wurden mit der feinen Kammseite der Naturkautschukkämme durchgeführt. Berechnet wurde die Restkämmarbeit pro Strähne wie folgt:

$$\text{Restkämmarbeit} = \text{Kämmarbeit vor Produktbehandlung/Kämmarbeit nach Produktbehandlung}$$

Die Restkämmbarkeit ist der Wert "NK %".

Anschließend wurde über die Quotienten aller 10 Strähnen der Mittelwert und die Standardabweichung bestimmt.

Aus den oben genannten Beispielen konnten die folgenden Schlüsse gezogen werden. Die Rezeptur -08 zeigte eine deutlich bessere NK im Vergleich zur Rezeptur -04. Eine weitere Verbesserung der Nasskämmbarkeit konnte durch die Verwendung des Verdickers Arlypon® TT liquid erzielt werden (Rezeptur -07). Gleichzeitig konnte der viskositätserniedrigende Effekt der Solubilisatoren aufgehoben werden.
Die als vorteilhaft aufgefundene Kombination aus den hydrophilen Solubilisatoren plus Verdicker wurde zu einem Produkt formuliert, dem "Hydrophilen Conditioning Compound". Zum Klarstellen wurde dem Produkt eine geringe Menge Wasser zugegeben Dieser Compound setzte sich folgendermaßen zusammen:
Zusammensetzung des **"Hydrophilen Conditioning Compound"** (INCI-Nomenklatur):

55,6 % Eumulgin® CO 40: hydrogenated Castor Oil + 40 mol EO
27,8 % Cetiol® HE: Coco, Mono- and Diglyceride + 7 mol EO
8,3 % Arlypon® TT liquid: Trimethylolpropan + 120 EO/10 PO - Random - tri - Oleat 8,3 % Wasser entmineralisiert

Der konditionierende Effekt (NK-Verbesserung) des hydrophilen Compounds konnte auch bei Verwendung weiterer Conditionierpolymere in klaren und perglänzenden Systemen nachgewiesen werden. Dies zeigen die Beispiele der folgenden Tabelle (Zusammensetzung in Gew.-%).

| Beispiele 14-017- | 01 | 02 | 05 | 21 | 22 | 23 | 24 |
|---|---|---|---|---|---|---|---|
| Texapon® N 70 | 14,30 | 14,30 | 14,30 | 14,30 | 14,30 | 14,30 | 14,30 |

(fortgesetzt)

| Beispiele 14-017- | 01 | 02 | 05 | 21 | 22 | 23 | 24 |
|---|---|---|---|---|---|---|---|
| Dehyton® PK 45 | 5,40 | 5,40 | 5,40 | 5,40 | 5,40 | 5,40 | 5,40 |
| Salcare® SC 60 | 0,10 | - | - | 0,10 | - | - | - |
| Polymer JR 400 | - | 0,20 | - | - | 0,20 | - | - |
| Dehyquart® Guar HP | - | - | - | - | - | 0,20 | - |
| Dehyquart® Guar N | - | - | - | - | - | - | 0,20 |
| Dehyquart® Guar TC | - | - | 0,20 | - | - | - | - |
| Euperlan® PK 710 | - | - | - | 3,0 | 3,0 | 3,0 | 3,0 |
| Hydrophiler Compound | 2,0 | 2,0 | 2,0 | 2,0 | 2,0 | 2,0 | 2,0 |
| Parfum Cotton Touch | 0,50 | 0,50 | 0,50 | 0,50 | 0,50 | 0,50 | 0,50 |
| Na-Benzoat | 0,50 | 0,50 | 0,50 | 0,50 | 0,50 | 0,50 | 0,50 |
| Zitronensäure 50 % in Wasser | 0,72 | 0,70 | 0,78 | 0,84 | 0,77 | 0,82 | 0,75 |
| NaCl | 2,21 | 2,62 | 2,37 | 1,72 | 1,67 | 1,38 | 1,37 |
| Wasser | 74,27 | 73,78 | 73,95 | 71,64 | 71,66 | 71,90 | 71,98 |
| Viskosität (mPas) | 5000 | 6100 | 6300 | 6040 | 5680 | 5800 | 5400 |
| NK (%) | 58 | 63 | 64 | 51 | 64 | 47 | 66 |
| Standardabweichung | 6 | 3 | 5 | 3 | 4 | 3 | 4 |
| TK (%) | 105 | | | | | 62 | |

Die Rezeptur -23 zeigte auch eine erhebliche Verbesserung der Nasskämmbarkeit bei besonders stark geschädigtem Haar (3 fach gebleicht). Die NK der Formulierung -23 betrug 52 % (+- 4 %), dagegen die Placeboformulierung ohne den hydophilen Compound 101% (+- 12 %).
TK bedeutet Trockenkämmbarkeit. Der Vergleich zeigt einen deutlich besseren Wert von Beispiel 23 verglichen mit Beispiel 1.

Auch **Variationen in der Zusammensetzung des Hydrophilen Conditioning Compounds** zeigten ähnliche Ergebnisse bei der Verbesserung des Kämmbarkeit. Dies zeigen die Beispiele der folgenden Tabelle (Zusammensetzung in Gew.-%).

| Beispiele 14-017- | 29 | 30 | 31 | 32 |
|---|---|---|---|---|
| Texapon® N 70 | 14,30 | 14,30 | 14,30 | 14,30 |
| Dehyton® PK 45 | 5,40 | 5,40 | 5,40 | 5,40 |
| Polymer JR 400 | 0,20 | 0,20 | 0,20 | 0,20 |
| Dehyquart® Guar TC | - | - | - | - |
| Hydrophiler Compound (Alternative 1) | 2,00 | - | - | - |
| Hydrophiler Compound (Alternative 2) | - | 2,00 | - | - |
| Hydrophiler Compound (Alternative 3) | - | - | 2,00 | - |
| Hydrophiler Compound (Alternative 4) | - | - | - | 2,00 |
| Parfum Cotton Touch | 0,50 | 0,50 | 0,50 | 0,50 |
| Na-Benzoat | 0,50 | 0,50 | 0,50 | 0,50 |
| Zitronensäure 50 % in Wasser | 0,73 | 0,73 | 1,08 | 0,65 |
| NaCl | 2,50 | 2,23 | 1,56 | 1,18 |
| Wasser | 73,87 | 74,14 | 74,46 | 75,27 |

(fortgesetzt)

| Beispiele 14-017- | 29 | 30 | 31 | 32 |
|---|---|---|---|---|
| NK (%) | 62 | 60 | 61 | 57 |
| Standardabweichung | +-4,3 | +-4,3 | +-5,3 | +-3,3 |

Hydrophiler Compound Alternative 1:
- 8,3 % Arlypon® TT liquid
- 55,6 % Eumulgin® HRE 40
- 27,8 % Cetiol® 767
- 8,3 % Wasser entmineralisiert

Hydrophiler Compound Alternative 2:
- 8,3 % Arlypon® TT liquid
- 55,6 % Eumulgin® HRE 40
- 27,8 % Cremophor® WO7
- 8,3 % Wasser entmineralisiert

Hydrophiler Compound Alternative 3:
- 8,3 % Arlypon® TT liquid
- 55,6 % Eumulgin® HRE 60
- 27,8 % Cetiol® HE
- 8,3 % Wasser entmineralisiert

Hydrophiler Compound Alternative 4:
- 8,3% Arlypon® TT liquid
- 27,8% Eumulgin® HRE 40
- 55,6% Cetiol® HE
- 8,3% Wasser entmineralisiert

In Versuchen zur **mikrobiellen Stabilität des Zwischenprodukts (hydrophiler Conditioning Compound)** erwies sich ein Wassergehalt von 8,3 % als hoch in Bezug auf die Wasseraktivität. Daher wurde in einer Variation ein Teil des Wassers durch Glycerin ersetzt. Dabei wurden die 8,3 % Wasser auf 4,3 % Wasser reduziert und 4 % Glycerin zugegeben, womit die Wasseraktivität in einen mikrobiell unbedenklichen Bereich abgesenkt wurde.

Eine Veränderung der vorteilhaften Eigenschaften des Zwischenprodukts im Hinblick auf seinen Beitrag zur Konditionierwirkung daraus hergestellter Zusammensetzungen durch den partiellen Austausch des Wassers gegen Glycerin ist nicht zu erwarten.

Eine mögliche Zusammensetzung des Compounds lautet daher:

Eumulgin® CO 40 (55,6 %): hydrogenated Castor Oil + 40 mol EO
Cetiol® HE (27,8 %): Coco, Mono- and Diglyceride + 7 mol EO
Arlypon® TT liquid (8,3 %): Trimethylolpropan + 120 EO/10 PO - Random - tri - Oleat
Water entmineralisiert (4,3 %)
Glycerin (4,0 %)

**Weitere Beispiele**

Ein Merkmal der erfindungsgemäßen Zusammensetzung ist die Anwesenheit von NaCl. Dieses gewährleistet, dass der Koacervatmechanismus aus anionischem Tensid und kationischem Polymer in der Verdünnungsphase initiiert wird. Dabei kann das NaCl durch das Cotensid (beispielsweise Cocamidopropylbetain, welches häufig bereits herstellungsbedingt ca. 5-7,5 Gew.-% NaCl enthält) mit eingeschleppt und/oder separat zugegeben werden. Die beiden folgenden Beispiele zeigen eine statistisch relevante Verbesserung der Nasskämmarbeit in Abhängigkeit von der Verwendung von NaCl:

| Beispiele 14-017- | 66 | 67 |
|---|---|---|
| Texapon® N 70 | 20.00 | 20.00 |
| Polymer JR 400 | 0,20 | 0,20 |
| Hydrophiler Compound | 3,30 | 3,30 |
| Parfum Cotton Touch | 0,50 | 0,50 |
| Na-Benzoat | 0,50 | 0,50 |
| Zitronensäure 50 % in Wasser | 0,60 | 0,75 |
| NaCl | - | 1,85 |
| Wasser | 74,90 | 73,78 |

(fortgesetzt)

| Beispiele 14-017- | 66 | 67 |
|---|---|---|
| NK (%) | 88 | 75 |
| Standardabweichung | 10 | 8 |

**Patentansprüche**

1. Eine Zusammensetzung, die als Shampoo oder Haarspülung geeignet ist, enthaltend

   - mindestens ein Tensid,
   - optional mindestens ein Cotensid, welches von dem mindestens einem Tensid verschieden ist,
   - mindestens ein kationisches Polymer,
   - ethoxylierte Fettsäureglyceride,
   - ethoxyilierte Mono- und Diglycerinester,
   - Trimethylpropan-EO/PO-Trioleat,
   - NaCl,
   - optional Glycerin,
   - optional weitere in Shampoos oder Haarspülungen übliche Inhaltsstoffe, und
   - Wasser.

2. Die Zusammensetzung nach Anspruch 1, wobei diese Zusammensetzung
   10 bis 20 Gew.-% des mindestens einen Tensids enthält,
   0 bis 15 Gew.-% des mindestens einen Cotensids enthält,
   0,1 bis 5 Gew.-% des kationischen Polymers enthält,
   0,06 bis 3,5 Gew.-% ethoxylierte Fettsäureglyceride enthält,
   0,06 bis 2,5 Gew.-% ethoxyilierte Mono- und Diglycerinester enthält,
   0,03 bis 0,5 Gew.-% Trimethylpropan-EO/PO-Trioleat enthält,
   0,1 bis 5 Gew.-%, insbesondere 0,3 bis 3 Gew.-%, NaCl enthält,
   optional Glycerin enthält,
   optional weitere in Shampoos oder Haarspülungen übliche Inhaltsstoffe enthält, und
   zur Ergänzung auf 100 Gew.-% Wasser enthält.

3. Die Zusammensetzung nach Anspruch 1 oder 2, wobei das kationische Polymer ausgewählt wird aus der Gruppe bestehend aus einem kationisch modifizierten Cellulosederivat, PQ 10, PQ 67, einem kationisch modifizierte Guarderivat, Guar Hydroxypropyltrimonium Chlorid, einem kationischen Homo- oder Copolymer auf der Basis von Acrylamid, einem kationischen Homo- oder Copolymer auf der Basis von Vinylpyrrolidon, einem kationischen Homo- oder Copolymer auf der Basis von quaternisiertem Vinylimidazol und einem kationischen Homo- oder Copolymer auf der Basis von Methacrylaten.

4. Die Zusammensetzung nach einem der Ansprüche 1 bis 3, wobei das mindestens eine Tensid ein anionisches Tensid ist.

5. Die Zusammensetzung nach einem der Ansprüche 1 bis 3, wobei das mindestens eine Tensid ausgewählt wird aus der Gruppe bestehend aus einem Sulfat, einem ethoxilierten Sulfat, einem Sulfonat, einem Alkylpolyglycosid, einem Derivat eines Alkylpolyglycosids, einem Betain, einem Amphoacetat, einem Glutamat, einem Sulfosuccinat, einem Taurat, einem Glycinat und einem Isethionat.

6. Die Zusammensetzung nach einem der der Ansprüche 1 bis 5, wobei die Zusammensetzung weniger als 2 Gew.-% Polysiloxane enthält.

7. Die Zusammensetzung nach einem der der Ansprüche 1 bis 6, wobei die Zusammensetzung weniger als 2 Gew.-% an Stoffen enthält, die Emollients oder Wachse sind.

8. Ein Zwischenprodukt, das zur Herstellung der Zusammensetzung nach einem der der Ansprüche 1 bis 7 geeignet ist, enthaltend

- ethoxylierte Fettsäureglyceride,
- ethoxyilierte Mono- und Diglycerinester,
- Trimethylpropan-EO/PO-Trioleat,
- NaCl
- optional Wasser, und
- optional Glycerin.

**9.** Das Zwischenprodukt nach Anspruch 8 enthaltend

- 12 bis 70 Gew.-% ethoxylierte Fettsäureglyceride,
- 12 bis 50 Gew.-% ethoxyilierte Mono- und Diglycerinester,
- 6 bis 10 Gew.-% Trimethylpropan-EO/PO-Trioleat,
- NaCl
- optional Wasser, und
- optional Glycerin.

**10.** Das Zwischenprodukt nach Anspruch 8 enthaltend

- 55 bis 56 Gew.-% ethoxiliertes, hydriertes Rizinusöl mit 40 Ethylenoxideinheiten,
- 27 bis 28 Gew.-% ethoxilierte Kokosöl-Partialglyceride mit 7 Ethylenoxideinheiten,
- 8 bis 9 Gew.-% Trimethylpropan-EO/PO-Trioleat mit 120 Ethylenoxideinheiten (EO) und 10 Propylenoxideinheiten (PO),
- NaCl
- 4 bis 5 Gew.-% Wasser, und
- 3 bis 5 Gew.-% Glycerin.

**11.** Die Verwendung des Zwischenproduktes nach einem der Ansprüche 8 bis 10 zur Herstellung der Zusammensetzung nach einem der Ansprüche 1 bis 7.

**12.** Ein Verfahren zur Herstellung der Zusammensetzung nach einem der Ansprüche 1 bis 7 umfassend

- das Bereitstellen des Zwischenproduktes nach einem der Ansprüche 8 bis 10 und
- das in Kontaktbringen des Zwischenproduktes mit den übrigen Bestandteilen der Zusammensetzung.

**13.** Die Verwendung der Zusammensetzung nach einem der der Ansprüche 1 bis 7 zur Konditionierung von Haaren, bevorzugt von menschlichen Haaren.

**14.** Die Verwendung der Zusammensetzung nach Anspruch 13 zur Verbesserung der Kämmbarkeit, bevorzugt zur Verbesserung der Nasskämmbarkeit, von Haaren, bevorzugt von menschlichen Haaren.

**15.** Die Verwendung nach Anspruch 13 oder 14, wobei die Haare geschädigt und hydrophil sind.

## Claims

**1.** A composition suitable as a shampoo or hair conditioner, comprising

- at least one surfactant,
- optionally at least one cosurfactant which is different from the at least one surfactant,
- at least one cationic polymer,
- ethoxylated fatty acid glycerides,
- ethoxylated mono- and diglycerol esters
- trimethylpropane EO/PO trioleate,
- NaCl,
- optionally glycerol,
- optionally further ingredients customary in shampoos or hair conditioners, and
- water.

**2.** The composition as claimed in claim 1, wherein said composition
comprises 10% to 20% by weight of the at least one surfactant,
comprises 0% to 15% by weight of the at least one cosurfactant,
comprises 0.1% to 5% by weight of the cationic polymer,
comprises 0.06 to 3.5% by weight ethoxylated fatty acid glycerides,
comprises 0.06 to 2.5% by weight ethoxylated mono- and diglycerol esters,
comprises 0.03 to 0.5% by weight trimethylpropane EO/PO trioleate,
comprises 0.1 to 5% by weight, in particular 0.3 to 3% by weight, NaCl,
optionally comprises glycerol,
optionally comprises further ingredients customary in shampoos or hair conditioners, and
comprises water to make up to 100% by weight.

**3.** The composition as claimed in claim 1 or 2, wherein the cationic polymer is selected from the group consisting of a cationically modified cellulose derivative, PQ 10, PQ 67, a cationically modified guar derivative, guar hydroxypropyltrimonium chloride, a cationic homo- or copolymer based on acrylamide, a cationic homo- or copolymer based on vinyl pyrrolidone, a cationic homo- or copolymer based on quaternized vinyl imidazole and a cationic homo- or copolymer based on methacrylates.

**4.** The composition as claimed in any of claims 1 to 3, wherein the at least one surfactant is an anionic surfactant.

**5.** The composition as claimed in any of claims 1 to 3, wherein the at least one surfactant is selected from the group consisting of a sulfate, an ethoxylated sulfate, a sulfonate, an alkyl polyglycoside, a derivative of an alkyl polyglycoside, a betaine, an amphoacetate, a glutamate, a sulfosuccinate, a taurate, a glycinate and an isethionate.

**6.** The composition as claimed in any of of claims 1 to 5, wherein the composition comprises less than 2% by weight polysiloxanes.

**7.** The composition as claimed in any of of claims 1 to 6, wherein the composition comprises less than 2% by weight of substances which are emollients or waxes.

**8.** An intermediate suitable for preparing the composition as claimed in any of of claims 1 to 7, comprising

- ethoxylated fatty acid glycerides,
- ethoxylated mono- and diglycerol esters
- trimethylpropane EO/PO trioleate,
- NaCl,
- optionally water, and
- optionally glycerol.

**9.** The intermediate as claimed in claim 8 comprising

- 12 to 70% by weight ethoxylated fatty acid glycerides,
- 12 to 50% by weight ethoxylated mono- and diglycerol esters,
- 6 to 10% by weight trimethylpropane EO/PO trioleate,
- NaCl,
- optionally water, and
- optionally glycerol.

**10.** The intermediate as claimed in claim 8 comprising

- 55 to 56% by weight ethoxylated hydrogenated castor oil having 40 ethylene oxide units,
- 27 to 28% by weight ethoxylated coconut oil partial glycerides having 7 ethylene oxide units,
- 8 to 9% by weight trimethylpropane EO/PO trioleate having 120 ethylene oxide units (EO) and 10 propylene oxide units (PO),
- NaCl,
- 4 to 5% by weight water, and
- 3 to 5% by weight glycerol.

**11.** The use of the intermediate as claimed in any of claims 8 to 10 for preparing the composition as claimed in any of claims 1 to 7.

**12.** A method for preparing the composition as claimed in any of claims 1 to 7, comprising

- providing the intermediate as claimed in any of claims 8 to 10 and
- bringing the intermediate into contact with the other constituents of the composition.

**13.** The use of the composition as claimed in any of of claims 1 to 7 for conditioning hair, preferably human hair.

**14.** The use of the composition as claimed in claim 13 for improving the combability, preferably for improving the wet combability, of hair, preferably human hair.

**15.** The use as claimed in claim 13 or 14, wherein the hair is damaged and hydrophilic.

## Revendications

**1.** Composition, qui est appropriée en tant que shampooing ou après-shampoing, contenant :

- au moins un tensioactif,
- éventuellement au moins un co-tensioactif, qui est différent dudit au moins un tensioactif,
- au moins un polymère cationique,
- des glycérides d'acides gras éthoxylés,
- des esters de mono- et diglycérine éthoxylés,
- du trioléate de triméthylpropane-EO/PO,
- du NaCl,
- éventuellement de la glycérine,
- éventuellement d'autres composants usuels dans les shampoings ou les après-shampoings, et
- de l'eau.

**2.** Composition selon la revendication 1, dans laquelle cette composition
contient 10 à 20 % en poids dudit au moins un tensioactif,
contient 0 à 15 % en poids dudit au moins un co-tensioactif,
contient 0,1 à 5 % en poids du polymère cationique,
contient 0,06 à 3,5 % en poids de glycérides d'acides gras éthoxylés,
contient 0,06 à 2,5 % en poids d'esters de mono- et diglycérine éthoxylés,
contient 0,03 à 0,5 % en poids de trioléate de triméthylpropane-EO/PO,
contient 0,1 à 5 % en poids, notamment 0,3 à 3 % en poids, de NaCl,
contient éventuellement de la glycérine,
contient éventuellement d'autres composants usuels dans les shampoings ou les après-shampoings,
et
contient de l'eau pour compléter jusqu'à 100 % en poids.

**3.** Composition selon la revendication 1 ou 2, dans laquelle le polymère cationique est choisi dans le groupe constitué par un dérivé de cellulose modifié cationiquement, PQ 10, PQ 67, un dérivé de guar modifié cationiquement, le chlorure de guar hydroxypropyltrimonium, un homo- ou copolymère cationique à base d'acrylamide, un homo- ou copolymère cationique à base de vinylpyrrolidone, un homo- ou copolymère cationique à base de vinylimidazole quaternisé et un homo- ou copolymère cationique à base de méthacrylates.

**4.** Composition selon l'une quelconque des revendications 1 à 3, dans laquelle ledit au moins un tensioactif est un tensioactif anionique.

**5.** Composition selon l'une quelconque des revendications 1 à 3, dans laquelle ledit au moins un tensioactif est choisi dans le groupe constitué par un sulfate, un sulfate éthoxylé, un sulfonate, un polyglycoside d'alkyle, un dérivé d'un polyglycoside d'alkyle, une bétaïne, un amphoacétate, un glutamate, un sulfosuccinate, un taurate, un glycinate et un iséthionate.

6. Composition selon l'une quelconque des des revendications 1 à 5, dans laquelle la composition contient moins de 2 % en poids de polysiloxanes.

7. Composition selon l'une quelconque des des revendications 1 à 6, dans laquelle la composition contient moins de 2 % en poids de substances qui sont des émollients ou des cires.

8. Produit intermédiaire, qui est approprié pour la fabrication de la composition selon l'une quelconque des des revendications 1 à 7, contenant :

- des glycérides d'acides gras éthoxylés,
- des esters de mono- et diglycérine éthoxylés,
- du trioléate de triméthylpropane-EO/PO,
- du NaCl,
- éventuellement de l'eau, et
- éventuellement de la glycérine.

9. Produit intermédiaire selon la revendication 8, contenant :

- 12 à 70 % en poids de glycérides d'acides gras éthoxylés,
- 12 à 50 % en poids d'esters de mono- et diglycérine éthoxylés,
- 6 à 10 % en poids de trioléate de triméthylpropane-EO/PO,
- du NaCl,
- éventuellement de l'eau, et
- éventuellement de la glycérine.

10. Produit intermédiaire selon la revendication 8, contenant :

- 55 à 56 % en poids d'huile de ricin hydrogénée éthoxylée contenant 40 unités oxyde d'éthylène,
- 27 à 28 % en poids de glycérides partiels d'huile de coco éthoxylés contenant 7 unités oxyde d'éthylène,
- 8 à 9 % en poids de trioléate de triméthylpropane-EO/PO contenant 120 unités oxyde d'éthylène (EO) et 10 unités oxyde de propylène (PO),
- du NaCl,
- 4 à 5 % en poids d'eau, et
- 3 à 5 % en poids de glycérine.

11. Utilisation du produit intermédiaire selon l'une quelconque des revendications 8 à 10 pour la fabrication de la composition selon l'une quelconque des revendications 1 à 7.

12. Procédé de fabrication de la composition selon l'une quelconque des revendications 1 à 7, comprenant :

- la préparation du produit intermédiaire selon l'une quelconque des revendications 8 à 10, et
- la mise en contact du produit intermédiaire avec les autres constituants de la composition.

13. Utilisation de la composition selon l'une quelconque des des revendications 1 à 7 pour le conditionnement de cheveux, de préférence de cheveux humains.

14. Utilisation de la composition selon la revendication 13 pour améliorer l'aptitude au démêlage, de préférence pour améliorer l'aptitude au démêlage à l'état humide, de cheveux, de préférence de cheveux humains.

15. Utilisation selon la revendication 13 ou 14, dans laquelle les cheveux sont endommagés et hydrophiles.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102008034388
- WO 0064410 A
- WO 2005048971 A
- FR 2252840 A